# EUROPÄISCHE PATENTSCHRIFT

(11) **EP 4 285 975 B1**
(45) Veröffentlichungstag und Bekanntmachung des Hinweises auf die Patenterteilung: **27.08.2025**
(21) Anmeldenummer: 23174850.0
(22) Anmeldetag: 23.05.2023
(51) Int. Cl.: A61M 16/00

(54) **BEATMUNGSGERÄT MIT ZWEI SERIELLEN GEBLÄSEN**
VENTILATOR WITH TWO SERIAL BLOWERS
APPAREIL RESPIRATOIRE À DEUX VENTILATEURS EN SÉRIE

(30) Priorität: 01.06.2022 DE 102022113882
(43) Veröffentlichungstag der Anmeldung: 06.12.2023
(73) Patentinhaber: Löwenstein Medical Technology S.A., 2557 Luxembourg (LU)
(72) Erfinder: Borm, Petrus Johannes Josephus, 5627 HP Eindhoven (NL)
(74) Vertreter: Löwenstein Medical IP

(56) Entgegenhaltungen:
- EP-A1- 3 501 583
- US-A1- 2007 199 566
- US-A1- 2018 043 116
- US-A1- 2021 290 876
- US-A1- 2022 143 353
- US-B2- 9 408 991

## Beschreibung

Die Offenbarung betrifft ein Beatmungsgerät mit zwei Gebläsen und ein Verfahren zum Betreiben eines Beatmungsgerätes mit zwei Gebläsen.

Im Stand der Technik sind Beatmungsgeräte mit einem Gebläse bekannt, über welches nur in eine Richtung Druck aufgebaut werden kann. Im Regelfall wird durch dieses eine Gebläse ein Überdruck erzeugt, welcher zur Beatmung von Patienten verwendet wird. Es sind ferner komplizierte pneumatische Anordnungen bekannt, bei denen durch eine Schaltung von verschiedenen Ventilen auch ein Unterdruck erzeugt werden kann. Diese fordern aber eine hohe Komplexität der Steuerung um die große Anzahl an Ventilen richtig zu steuern. Eine solche Umschaltung ermöglicht auch nur eine plötzliche Veränderung der Druckverhältnisse, der gerade herrschende Überdruck wird beim Schalten der Ventile also direkt in einen ebenso großen Unterdruck geändert.

Die US2021/290876 A1 offenbart ein Beatmungsgerät mit einem Steuermodul und zwei Gebläsen zur Erzeugung eines Überdrucks und/oder eines Unterdrucks und zumindest einen Anschluss zur Verbindung mit einem Schlauch. Das Steuermodul umfasst eine Steuereinheit zur Steuerung der Gebläse.

Die folgenden Dokumente offenbaren weitere Beatmungsgeräte mit mindestens zwei Gebläsen, nämlich US 2018/043116 A1, US 2007/199566 A1, EP 3 501 583 A, US 2022/143353 A and US 9 408 991 B2.

Die Erfindung ist in den angehängten Ansprüchen definiert. Aufgabe der vorliegenden Offenbarung ist daher, ein Beatmungsgerät bereitzustellen, welches sowohl eine Überdruck- als auch eine Unterdruckbeatmung bietet.Die Offenbarung betrifft ein Beatmungsgerät zur Beatmung eines Lebewesens umfassend zumindest ein Gasmodul und ein Steuermodul, wobei das Gasmodul zumindest zwei Gebläse zur Erzeugung eines Überdrucks und/oder eines Unterdrucks und zumindest einen Anschluss zur Verbindung mit einem Schlauch umfasst und das Steuermodul zumindest eine Steuereinheit zur Steuerung des Gasmoduls umfasst. Das Beatmungsgerät kennzeichnet, dass zumindest ein erstes Gebläse eine dem zumindest zweiten Gebläse entgegengesetzte Förderrichtung aufweist. Die Steuereinheit ist eingerichtet und ausgebildet die Gebläse so anzusteuern, dass zumindest ein erstes Gebläse eine dem zumindest zweiten Gebläse entgegengesetzte Förderrichtung aufweist, wobei ein Unterdruck in den Atemwegen des Patienten beziehungsweise im Anschluss erzeugt werden.

In manchen Ausführungsformen kennzeichnet das Beatmungsgerät, dass die zumindest zwei Gebläse über ihre Auslassstutzen miteinander verbunden sind.

In manchen Ausführungsformen kennzeichnet das Beatmungsgerät, dass die zumindest zwei Gebläse über ihre Ansaugstutzen miteinander verbunden sind.

In manchen Ausführungsformen kennzeichnet das Beatmungsgerät, dass zumindest ein erstes Gebläse so angeordnet ist, dass das Gebläse auf der Seite des Anschlusses zumindest zweitweise einen Überdruck erzeugt und zumindest ein zweites Gebläse so angeordnet ist, dass das Gebläse auf der Seite des Anschlusses zumindest zeitweise einen Unterdruck erzeugt.

In manchen Ausführungsformen kennzeichnet das Beatmungsgerät, dass bei der Erzeugung eines Überdrucks auf Seiten des Anschlusses durch zumindest eines der Gebläse, das zumindest eine andere Gebläse mit entgegengesetzter Förderrichtung stillsteht und/oder auf einer Drehzahl läuft, welche geringer ist als die des einen Gebläses und/oder einen geringeren Druck erzeugt als das eine Gebläse.

In manchen Ausführungsformen kennzeichnet das Beatmungsgerät, dass bei der Erzeugung eines Unterdrucks auf Seiten des Anschlusses durch das andere Gebläse, das zumindest eine Gebläse mit entgegengesetzter Förderrichtung stillsteht und/oder auf einer Drehzahl läuft, welche geringer ist als die des anderen Gebläses und/oder einen geringeren Druck erzeugt als das andere Gebläse.

In manchen Ausführungsformen kennzeichnet das Beatmungsgerät, dass zumindest eins der Gebläse dazu eingerichtet ist einen Unterdruck und/oder negativen Druck zu erzeugen.

In manchen Ausführungsformen kennzeichnet das Beatmungsgerät, dass das Beatmungsgerät dazu eingerichtet ist, eine Beatmung mit negativem Exspirationsdruck bereitzustellen.

In manchen Ausführungsformen kennzeichnet das Beatmungsgerät, dass das Beatmungsgerät dazu eingerichtet ist, einen konstanten Exspirationsfluss zu erzeugen, wobei im Laufe der Exspiration über eins der Gebläse ein negativer Exspirationsdruck bereitgestellt wird.

In manchen Ausführungsformen kennzeichnet das Beatmungsgerät, dass das Beatmungsgerät dazu eingerichtet ist, eine Hustentherapie durchzuführen, wobei einem Patienten während der Inspiration durch das eine Gebläse ein Atemgas mit Überdruck bereitgestellt wird und nach dem Ende der Inspiration ein schnelles Umschalten zu einem, durch das andere Gebläse zur Verfügung gestellte, Atemgas mit Unterdruck gewechselt wird.

In manchen Ausführungsformen kennzeichnet das Beatmungsgerät, dass das Beatmungsgerät dazu eingerichtet ist, eine überlagerte und/oder oszillierende Beatmung bereitzustellen.

In manchen Ausführungsformen kennzeichnet das Beatmungsgerät, dass das eine Gebläse die Beatmungsfrequenz bestimmt und das andere Gebläse die überlagerte Frequenz erzeugt.

In manchen Ausführungsformen kennzeichnet das Beatmungsgerät, dass das Beatmungsgerät dazu eingerichtet ist, gleichzeitig eine überlagerte Beatmung und eine Beatmung mit negativem Exspirationsdruck bereitzustellen.

In manchen Ausführungsformen kennzeichnet das Beatmungsgerät, dass zwischen dem Anschluss und den Gebläsen zumindest eine Gasquelle angeordnet ist, wobei zumindest ein Gas aus der Gasquelle über zumindest ein Ventil in das durch eins der Gebläse geförderte Atemgas eingeleitet wird.

In manchen Ausführungsformen kennzeichnet das Beatmungsgerät, dass im Bereich des Ventils ein Mischbereich zur besseren Durchmischung von gefördertem Atemgas und durch das Ventil eingeleitete Gas angeordnet ist.

Es ist darauf hinzuweisen, dass die in den Ansprüchen einzeln aufgeführten Merkmale in beliebiger, technisch sinnvoller Weise miteinander kombiniert werden können und weitere Ausgestaltungen der Offenbarung aufzeigen. Die Beschreibung charakterisiert und spezifiziert die Offenbarung insbesondere im Zusammenhang mit den Figuren zusätzlich.

Es sei ferner darauf hingewiesen, dass eine hierin verwendete, zwischen zwei Merkmalen stehende und diese miteinander verknüpfende Konjunktion "und/oder" stets so auszulegen ist, dass in einer ersten Ausgestaltung des offenbarungsgemäßen Gegenstands lediglich das erste Merkmal vorhanden sein kann, in einer zweiten Ausgestaltung lediglich das zweite Merkmal vorhanden sein kann und in einer dritten Ausgestaltung sowohl das erste als auch das zweite Merkmal vorhanden sein können.

Unter einem Beatmungsgerät ist jedwedes Gerät zu verstehen, welches einen Anwender oder Patienten bei der natürlichen Atmung unterstützt, die Beatmung des Anwenders bzw. Lebewesens (z.B. Patient und/oder Neugeborener und/oder Frühgeborene) übernimmt und/oder zur Atemtherapie dient und/oder anderweitig die Atmung des Anwenders bzw. Patienten beeinflusst. Darunter fallen zum Beispiel, aber nicht ausschließend, CPAP- sowie BiLevel-Geräte, Narkose- bzw. Anästhesiegeräte, Atemtherapiegeräte, (klinische, außerklinische oder Notfall-) Beatmungsgeräte, Highflow-Therapiegeräte und Hustenmaschinen. Beatmungsgeräte können auch als Diagnosegeräte für die Beatmung verstanden werden. Diagnosegeräte können dabei allgemein zur Erfassung von medizinischen und/oder atmungsbezogenen Parametern eines Lebewesens dienen. Darunter fallen auch Geräte, welche medizinische Parameter von Patienten in Kombination mit der Atmung oder ausschließlich die Atmung betreffend, erfassen und optional verarbeiten können, wie beispielsweise Simulatoren.

Als Patienteninterface kann, soweit nicht ausdrücklich anders beschrieben, jegliches Peripheriegerät verstanden werden, welches zur Interaktion, insbesondere zu Therapie- oder Diagnosezwecken, der Messeinrichtung mit einem Lebewesen gedacht ist. Insbesondere kann ein Patienteninterface als Maske eines Beatmungsgerätes bzw. eine mit dem Beatmungsgerät verbundene Maske verstanden werden. Diese Maske kann eine Full-Face Maske, also Nase und Mund umschließende, oder eine Nasenmaske, also eine nur die Nase umschließende Maske, sein. Auch Trachealtuben bzw. -kanülen und sogenannte Nasenbrillen können als Maske beziehungsweise Patienteninterface eingesetzt werden. In manchen Fällen kann das Patienteninterface auch ein einfaches Mundstück, beispielsweise ein Rohr, sein, durch welches das Lebewesen zumindest ausatmet und/oder einatmet.

Im Zuge der Beschreibung sind Beatmung und Therapie als Synonyme zu verstehen, soweit nicht explizit anders angegeben. Stellenweise kann unter einer Therapie auch die einmalige und/oder wiederkehrende Durchführung eines Manövers, z.B. eines Rekrutierungsmanövers und/oder eines Hustenmanövers, verstanden werden.

Das erfindungsgemäße Beatmungsgerät ist dadurch charakterisiert, dass zumindest zwei Gebläse in Serie geschaltet werden, wobei zumindest zwei Gebläse eine entgegengesetzte Förderrichtung aufweisen. Über eine solche Anordnung können mehrere fortgeschrittene Beatmungs- bzw. Therapieformen angeboten werden. Beispielsweise kann das Beatmungsgerät neben einem Beatmungsmodus auch als Hustenmaschine (Insufflation/Exsufflation) eingesetzt werden bzw. zur Unterstützung des Hustens. Auch ermöglicht es einen negativen Exspirationsdruck während der Beatmung. Es kann weiter vorgesehen sein, dass auch eine überlagerte Beatmung und/oder eine hochfrequent oszillierende Beatmung möglich ist.

In manchen Ausführungsformen sind Gebläse vorgesehen, welche innerhalb kürzester Zeit eine hohe Drehzahl und/oder Fluss und/oder Druck erreichen können. Bei dem Einsatz von solchen hoch-reaktiven Gebläsen können in manchen Ausführungsformen zum Teil Ventile eingespart werden und die Reaktionszeit des Beatmungsgerätes teilweise zusätzlich verbessert werden.

In manchen Ausführungsformen ist im Gasmodul ein zusätzlicher Gasweg vorgesehen, um ein 2-Schlauchsystem mit dem System zu verbinden, wobei je nach Atemphase über ein oder mehrere Ventile der inspiratorische oder der exspiratorische Zweig geöffnet bzw. geschlossen wird.

In manchen Ausführungsformen ist vorgesehen, dass zusätzliche Steuerventil angeordnet sind, etwa um ein Schlauchsystem mit Patientenventil steuern zu können. Der Steuerdruck kann dabei beispielsweise ebenfalls von zumindest einem der Gebläse erzeugt werden.

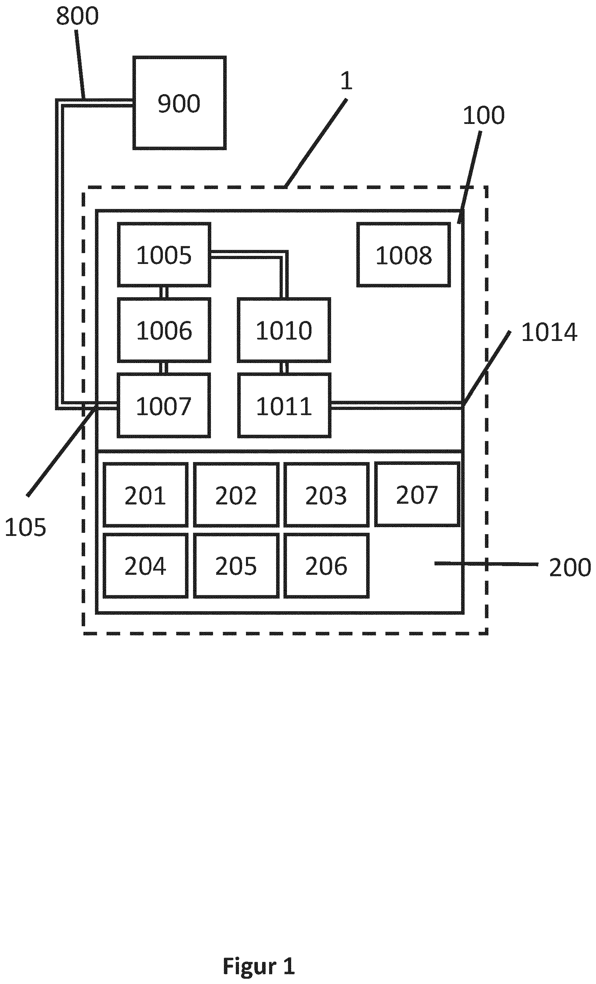

Die Offenbarung wird anhand der Figuren 1 bis 3 beispielhaft näher beschrieben.

Figur 1 zeigt eine schematische Darstellung einer beispielhaften Ausführungsform des Beatmungsgerätes 1. Beispielhaft unterteilt sich das Beatmungsgerät 1 in ein Gasmodul 100 und ein Steuermodul 200. Das Steuermodul ist dazu eingerichtet und ausgebildet das Gasmodul 100 zu steuern.

Beispielhaft ist das Beatmungsgerät 1 über eine Verbindung 800 mit einem Patienten 900 verbunden. Über die Verbindung 800 kann eine gasleitende Verbindung zwischen dem Beatmungsgerät 1 und dem Patienten 900 hergestellt werden, beispielsweise über den Anschluss 105 des Gasmoduls 100. Die Verbindung 800 kann beispielsweise als Beatmungsschlauch und/oder Schlauchsystem ausgeführt sein. Die Verbindung zum Patienten 900 wird etwa durch ein Patienteninterface, zum Beispiel eine Beatmungsmaske oder ein Tubus, erreicht.

Das Gasmodul 100 des Beatmungsgeräts 1 umfasst beispielhaft ein Gebläse 1010 zur Erzeugung eines Überdrucks in den Atemwegen des Patienten, ein Gebläse 1011 zur Erzeugung eines Unterdrucks in den Atemwegen des Patienten sowie Sensorik zur Bestimmung der Gasparameter innerhalb des Gasmoduls 100. Durch das Gebläse 1010 ist das Beatmungsgerät 1 dazu eingerichtet und ausgebildet eine Überdruckbeatmung des Patienten 900 durchzuführen, um die Atmung des Patienten 900 zu unterstützen und/oder den Patienten 900 zu beatmen, also im Wesentlichen die Beatmung des Patienten 900 vorzugeben. Das Gebläse 1010 ist zudem dazu eingerichtet beispielsweise Umgebungsluft als Atemgas anzusaugen und in Richtung des Patienten 900 zu fördern. Es kann auch vorgesehen sein, dass ein künstliches Atemgas, beispielsweise aus Druckgasflaschen oder einer Druckgasleitung, bereitgestellt wird.

An dieser Stelle ist anzumerken, dass Gebläse zwar in eine Richtung einen Druck aufbauen können, durch ein Gebläse aber durchaus in zwei Richtungen der Gasfluss erfolgen kann. Damit über das Gebläse 1010 ein Gasfluss in die Druckrichtung, also in Richtung Patienten 900, erfolgt, muss das Gebläse 1010 den Gegendruck - erzeugt beispielsweise durch den Patienten 900 - überwinden, also einen höheren Druck bereitstellen als der Patient 900.

Die Sensorik, zum Beispiel umfassend zumindest einen Drucksensor 1005, einen Flusssensor 1006, einen Temperatursensor 1007 ist dazu eingerichtet und ausgebildet, Messwerte des Gases im Gasmodul 100 zu erfassen. Die Messwerte betreffen beispielsweise Druck, Fluss, Temperatur, Feuchtigkeit und/oder Gaszusammensetzung. Die Sensorik ist beispielsweise zwischen den Gebläse 1010, 1011 und dem Anschluss 105 angeordnet. Über die Sensorik können unter anderem Rückschlüsse auf die Atemparameter wie beispielsweise Frequenz, Druck, Fluss des Patienten 900 getroffen werden. Es kann auch vorgesehen, dass anhand der von der Sensorik erfassten Messwerte verschiedene Atemsituationen wie etwa Apnoen, Schnarchen, Schlaf-/Wachzustand, normale Atmung, etc. erkannt werden können. In manchen Ausführungsformen ist das Steuermodul 200 beispielhaft dazu eingerichtet und ausgebildet die Beatmungsparameter entsprechend der jeweiligen Atemsituation anzupassen.

Zur Steuerung des Gasmoduls 100 umfasst das Steuermodul 200 eine Steuereinheit 201, welche dazu ausgebildet ist zumindest die Gebläse 1010 und 1011 zu steuern. Die Steuereinheit 201 ist ferner dazu eingerichtet und ausgebildet auf Grundlage von Vorgaben und/oder Einstellungen und/oder Berechnungen zur Beatmung und/oder Atmung des Patienten 900 das Gasmodul 100 anzusteuern.

Die Steuerung des Gasmoduls 100 wird beispielhaft über das Steuermodul 200 gewährleistet. Dazu umfasst das Steuermodul 200 zumindest eine Steuereinheit 201, welche dazu eingerichtet ist zumindest die beiden Gebläse 1010, 1011 anzusteuern. Die Steuerung kann beispielsweise anhand von Vorgaben erfolgen. In manchen Ausführungsformen kann vorgesehen sein, dass das Beatmungsgerät 1 auf die Beatmungssituation des Patienten 900 reagiert. Dazu kann das Steuermodul 200 beispielhaft eine Berechnungseinheit 202 umfassen, welche dazu eingerichtet ist, die zu erreichende Beatmung anhand der Vorgaben und/oder aufgenommener Sensorwerte zu bestimmen.

Das Steuermodul 200 des Beatmungsgeräts 1 umfasst zudem beispielhaft eine Sensoreinheit 204, eine Auswerteeinheit 203, eine Eingabeeinheit 205 sowie eine Speichereinheit 206. Die Sensoreinheit 204 ist dazu eingerichtet und ausgebildet die über die Sensorik erfassten Messwerte aufzunehmen und gegebenenfalls aufzubereiten. Die Auswerteeinheit 203 ist dazu eingerichtet und ausgebildet, die von der Sensoreinheit 204 aufgenommenen und optional aufbereiteten Messwerte auszuwerten und/oder zu analysieren. Beispielsweise kann vorgesehen sein, dass die Auswerteeinheit 203 die Messwerte dahingehend analysiert, ob die vorgegebene Steuerung des Gasmoduls 100 korrekt erfolgt, beispielsweise die gewünschten Drücke, Flüsse und/oder Volumina erzeugt werden. Auch kann vorgesehen sein, dass die Auswerteeinheit 203 dazu ausgebildet und eingerichtet ist, die Atmungsparameter des Patienten 900 anhand der Messwerte zu ermitteln. Es kann auch vorgesehen sein, dass die Auswerteeinheit 203 anhand der Messwerte bestimmte Atmungssituationen erkennt. Die Ergebnisse der Analyse und/oder Auswertung werden beispielsweise über die Eingabeeinheit 205 zur Berechnungseinheit 202 weitergeleitet. Über die Berechnungseinheit 202 können die Analyseergebnisse und/oder auch die Messwerte mit in die Bestimmung der Beatmungsparameter einbezogen werden, welche die Grundlage der Steuerung des Gasmoduls 100 bilden.

Beispielhaft umfasst das Steuermodul 200 eine Speichereinheit 206. In der Speichereinheit 206 können Messwerte, Analysen und/oder Auswertungen zumindest zwischengespeichert werden. In manchen Ausführungsformen des Beatmungsgeräts 1 ist vorgesehen, dass die durch die Sensorik erfassten Messwerte in der Speichereinheit 206 abgespeichert werden.

Die Eingabeeinheit 205 dient bespielhaft als Schnittstelle, über welche Daten, Werte und/oder Informationen in das Beatmungsgerät 1, insbesondere in das Steuermodul 200, eingegeben werden können. In manchen Ausführungsformen erfolgt auch eine systeminterne Eingabe von Daten, Werten und/oder Informationen über die Eingabeeinheit 205, beispielsweise aus der Auswerteeinheit 203, an die Berechnungseinheit 202. Es ist auch angedacht, dass die Eingabeeinheit 205 dazu eingerichtet und ausgebildet ist, Daten, Werte und/oder Informationen an ein externes Gerät weiterzugeben. Beispielsweise kann dazu über eine Schnittstelle eine entfernte Gegenstelle, etwa ein Computer, Notebook, Smartphone, Server, eine Cloud und/oder ein Tablet mit dem Beatmungsgerät 1 verbunden sein. Alternativ oder ergänzend kann auch eine Benutzerschnittstelle 207 am Beatmungsgerät 1 vorgesehen sein, welche beispielhaft dazu ausgebildet ist, Daten/Werte/Informationen bezüglich der Beatmung anzuzeigen und auch dazu eingerichtet sein kann, dass ein Nutzer Daten/Werte/Informationen eingeben kann. Die Benutzerschnittstelle 207 ist insbesondere dazu eingerichtet, Vorgaben und/oder Einstellungen bezüglich der Beatmung in das Beatmungsgerät 1 einzugeben. Die Berechnungseinheit 202 ist beispielsweise dazu eingerichtet und ausgebildet, anhand der Vorgaben und/oder Einstellungen die Beatmungsparameter zu bestimmen. Die Vorgaben und/oder Einstellungen umfassen beispielsweise aber nicht ausschließend Druck, Fluss, Lungenvolumen, Gaszusammensetzung, Atemfrequenz, Atemzugsvolumen, Art des Lebewesens, Alter, Gewicht, Krankheiten (insbesondere respiratorische Krankheiten), Gasaustausch, Atemprobleme. In manchen Ausführungsformen ist vorgesehen, dass die Benutzerschnittstelle 207 über eine Eingabemaske verfügt, über welche Einstellungen bezüglich der Beatmung eingegeben werden, welche an das Steuermodul 200 übermittelt werden. In manchen Ausführungsformen sind in der Speichereinheit 206 eine Vielzahl an Beatmungsvorlagen und/oder Beatmungsprogrammen hinterlegt, auf welche über die Benutzerschnittstelle 207 zugegriffen werden kann.

Im Zuge der Analyse der Messwerte durch die Auswerteeinheit 203 kann auch vorgesehen sein, dass die Steuereinheit 201 und/oder die Berechnungseinheit 202 selbstständig auf vorprogrammierte Beatmungsvorlagen zugreifen kann und diese zur Beatmung des Patienten 900 ausführen kann.

In manchen Ausführungsformen ist es vorgesehen, dass eine Anzeige der aktuellen Beatmung, beispielsweise in Form von Werten und/oder Graphen, über die Benutzerschnittstelle 300 möglich ist.

Zur Förderung des Atemgases sind in dem Gasmodul 100 zwei Gebläse 1010, 1011 angeordnet. Alternativ oder ergänzend zu den zwei Gebläsen 1010, 1011 kann auch zumindest eine bidirektionale Pumpe eingesetzt werden.

Die Förderrichtung der Gebläse 1010, 1011 ist dabei entgegengesetzt, sodass sowohl ein Überdruck als auch ein Unterdruck auf Seite des Anschlusses 105 erzeugt werden kann. Beispielsweise fungiert das Gebläse 1010 zur Erzeugung eines Überdrucks. Mit Förderrichtung ist dabei insbesondere die Druckrichtung gemeint. Beispielsweise baut das Gebläse 1010 einen Druck in Richtung Anschluss 105 bzw. Patienten 900 auf. Das Gebläse 1010 saugt dazu beispielsweise Gas über den Auslass 1014, welcher alternativ oder ergänzend als Ansaugbereich ausgebildet sein kann, an und fördert das Gas durch das Gasmodul 100 zum Anschluss 105. Ein Unterdruck wird beispielsweise über das Gebläse 1011 erzeugt, welches dazu eingerichtet ist entgegen der Förderrichtung des Gebläses 1010 Gas zu fördern. Durch die entgegengesetzte Förderrichtung, also ein Ansaugen von Gas am Anschluss 105, kann beispielsweise ein negativer Exspirationsdruck beim Patienten 900 erzeugt werden.

Über die Steuereinheit 201 lassen sich insbesondere die Gebläse 1010 und 1011 ansteuern. Beispielsweise wird zur Erzeugung eines Unterdrucks ausschließlich das Gebläse 1011 aktiviert, welches so angeordnet ist, dass Gas durch den Anschluss 105 ansaugt. Gleichermaßen kann vorgesehen sein, dass zur Erzeugung eines Überdrucks ausschließlich das Gebläse 1010 aktiviert wird, welches Gas von dem Auslass 1014 ansaugt. Alternativ oder ergänzend kann vorgesehen sein, dass beide Gebläse 1010, 1011 dauerhaft aktiv sind und auf einem Drehzahlniveau und/oder Druckniveau laufen. In manchen Ausführungsformen kann es vorteilhaft sein, dass beide Gebläse gegeneinander arbeiten um einen besseren Arbeitspunkt für die Gebläse zu erreichen. Auch kann vorgesehen sein, dass je nach Beatmungs- bzw. Therapieform die Gebläse 1010, 1011 aktiviert oder deaktiviert werden. Ist beispielsweise vorgesehen, dass eine Hustentherapie gestartet wird, so kann das Gebläse 1011 für den Zeitraum der Hustentherapie aktiviert werden und nach Beendigung wieder deaktiviert werden. Während der Therapie läuft das Gebläse 1011 in den Zeiträumen, in denen kein Unterdruck erzeugt werden soll, also auf einer bestimmten Drehzahl, wobei die Drehzahl erhöht wird um einen Unterdruck zu erzeugen.

Die Gebläse 1010, 1011 sind beispielsweise so eingerichtet und angeordnet, dass Gas ungehindert entgegen der Förderrichtung durch die Gebläse strömen kann ohne diese, beispielsweise durch erzwungene Rotation der Förderräder entgegen der angedachten Richtung, beschädigt werden. In manchen Ausführungsformen ist vorgesehen, dass Bypass-Leitungen in dem Gasmodul 100 so angeordnet sind, dass Gas an den Gebläsen vorbeiströmen kann, während das jeweilige Gebläse deaktiviert ist.

Während die Steuereinheit 201 dazu eingerichtet ist, die Gebläse 1010, 1011 so anzusteuern, dass die Vorgaben zur Beatmung erreicht werden, wird die dadurch umgesetzte Beatmung über die Sensorik, beispielhaft über den Drucksensor 1005, den Flusssensor 1006 und den Temperatursensor 1007, überprüft. Die Auswerteeinheit 203 ist beispielsweise dazu eingerichtet und ausgebildet, die Messerwerte der Sensoren 1005, 1006, 1007 auszuwerten und daraufhin zu analysieren, ob die Beatmung entsprechend der Vorgaben durchgeführt wird.

Beispielsweise ist die Berechnungseinheit 202, gegebenenfalls in Kombination mit der Auswerteeinheit 203, dazu eingerichtet und ausgebildet, die Beatmung mit der Atmung des Patienten 900 zu vergleichen und auf Abweichungen zu überprüfen. In manchen Ausführungsformen ist die Berechnungseinheit 202 und/oder die Steuereinheit 201 dazu eingerichtet, etwaige Korrekturen der Beatmung automatisch durchzuführen. Die Berechnungseinheit 202 ist beispielsweise auch dazu eingerichtet und ausgebildet, Gasparameter, beispielsweise Druck und/oder Fluss und/oder Temperatur und/oder Gaszusammensetzung, mit in die Bestimmung der Beatmungsparameter einzubeziehen. Beispielsweise wird bei der Bestimmung der Beatmungsparameter durch die Berechnungseinheit 202 der vom Patienten 900 erzeugte Druck und/oder Fluss mit einbezogen.

Über das Steuermodul 200, insbesondere die Steuereinheit 201, wird das Gasmodul 100 gesteuert. Die Steuersignale zur Steuerung werden zum Beispiel über die Berechnungseinheit 202 aus bestimmten Beatmungsparametern abgleitet. Über Benutzerschnittstelle 207 werden beispielsweise die Vorgaben zur Beatmung bzw. Therapie eingegeben. Die Vorgaben können zum Beispiel Lungenvolumen, Fluss, Druck, Atemzugsvolumen, Atemfrequenz, Beatmungs- bzw. Therapieprogramm, Größe, Gewicht, Erkrankungen, etc. des Patienten 900 betreffen. In manchen Ausführungsformen können zumindest Eingaben hinsichtlich Druck und Fluss der Beatmung bzw. Therapie gemacht werden.

In der Speichereinheit 206 können Vorlagen, beispielsweise für verschiedene Beatmungs- bzw. Therapieformen hinterlegt sein. Diese Vorlagen können beispielsweise angepasst werden. In manchen Ausführungsformen kann vorgesehen sein, dass die Beatmungsparameter und/oder Vorgaben auch während der Beatmung über die Benutzerschnittstelle 207 angepasst werden können, beispielsweise ohne die aktuelle Beatmung bzw. Therapie zu unterbrechen.

Entsprechend der Vorgaben erfolgt durch die Berechnungseinheit 202 in einem ersten Schritt die Bestimmung der Beatmungsparameter. Aus den Beatmungsparametern werden entsprechende Steuersignale abgeleitet und an die Steuereinheit 201 übermittelt. Anhand der Steuersignale steuert die Steuereinheit 201 in einem zweiten Schritt das Gasmodul 100, beispielsweise um den Patienten 900 zu beatmen, bei der Atmung zu unterstützen oder eine andere Therapieform durchzuführen, beispielsweise ein Hustenmanöver.

Durch die Verwendung von zwei Gebläsen in einem Beatmungsgerät ergeben sich zusätzliche Therapiemöglichkeiten durch ein einzelnes Gerät. Neben einer Überdruckbeatmung ist so auch ein negativer Exspirationsdruck möglich. Während eins der Gebläse 1010, 1011 einen Überdruck auf der Seite des Anschlusses 105 bzw. beim Patienten 900 erzeugen kann, ist durch die entgegengesetzte Druck- bzw. Förderrichtung des anderen Gebläses 1010, 1011 auch eine Erzeugung eines Unterdrucks auf der Seite des Anschlusses 105 bzw. beim Patienten 900 möglich.

Das Beatmungsgerät 1 ist beispielhaft auch dazu eingerichtet, eine Fluss-kontrollierte Beatmung mit einem negativen exspiratorischen Fluss und/oder Druck umzusetzen, wobei während der Inspiration ein konstanter Fluss vorgegeben wird und während der Exspiration ein konstanter Fluss vorgegeben wird. Das Verhältnis zwischen inspiratorischen und exspiratorischen Fluss ist beispielsweise 1, der Fluss während der Inspiration entspricht im Betrag also dem Fluss der Exspiration, wobei das Vorzeichen umgekehrt ist. Um einen konstanten, negativen Fluss während der Exspiration zu erzeugen ist vorgesehen, dass durch das zweite Gebläse 1011 ein Unterdruck erzeugt werden kann, sodass ein konstanter Fluss erreicht wird.

Bei einer natürlichen Exspiration nehmen der Fluss und der Druck in der Regel zum Ende der Exspiration ab, bis der Druck auf einem (positiven) Minimum angelangt ist. Durch eine über das zweite Gebläse 1011 unterstütze weitere, beispielsweise lineare, Druckverringerung hin zu negativen Drücken kann ein konstanter Exspirationsfluss erreicht werden.

In manchen Ausführungsformen ist das Beatmungsgerät 1 dazu ausgebildet eine Hustentherapie durchzuführen. Um dem Patienten 900 das Husten zu ermöglichen und/oder ein Husten beim Patienten 900 auszulösen und/oder die Hustenfunktion für den Patienten 900 zu übernehmen, wird dem Patienten über das Gebläse 1010 zunächst ermöglicht tief einzuatmen. Nach der Inspiration wird über ein schnelles Abbremsen des Gebläses 1010 und ein optional schnelles Beschleunigen des Gebläses 1011 das Husten durchgeführt. Durch die schnelle Umkehrung des Flusses und/oder des Druckes kann so beispielsweise Sekret aus den Atemwegen des Patienten 900 entfernt werden.

In manchen Ausführungsformen ist das Beatmungsgerät 1 dazu ausgebildet eine überlagerte Beatmung bereitzustellen. Dabei wird die Beatmungsfrequenz mit einer hohen Frequenz in Form von geringen Fluss- und/oder Druckänderungen überlagert. Es kann vorgesehen sein, dass ein Gebläse alleine diese Fluss- und/oder Druckänderungen erzeugt. In dem vorgestellten Beatmungsgerät 1 kann aber auch vorgesehen sein, dass zum Beispiel das Gebläse 1010 die Atemfrequenz erzeugt, also den Patienten 900 beatmet während das Gebläse 1011 die überlagerte Frequenz erzeugt. Die überlagerte Beatmung kann beispielsweise zusammen mit einem Trachealtubus genutzt werden, etwa um weitere Lungen- und Atmungsparameter bestimmen zu können. Alternativ oder ergänzend können über die überlagerte Beatmung auch Eigenschaften des und/oder im Tubus bestimmt werden. Durch die zwei Gebläse 1010, 1011 ist das Beatmungsgerät 1 auch dazu eingerichtet eine überlagerte Beatmung zusammen mit einem negativen Exspirationsdruck durchzuführen.

In manchen Ausführungsformen ist das Beatmungsgerät 1 ergänzend oder alternativ dazu ausgebildet eine hochfrequente Oszillation des Druckes zu erzeugen. Beispielsweise kann die hochfrequente Oszillation über einen PEEP (positiver endexspiratorischer Druck, positive endexspiratory pressure) gelegt werden. Während beispielhaft das Gebläse 1010 den PEEP bereitstellt kann das Gebläse 1011 durch periodische Änderungen der Drehzahl eine hochfrequente Oszillation des Druckes erzeugen. In manchen Ausführungsformen kann der PEEP zusammen mit der hochfrequenten Oszillation des Druckes durch ein einzelnes Gebläse erzeugt werden. Eine hohe Frequenz in diesem Sinne liegt beispielhaft über 3 Hz. In manchen Ausführungsformen kann vorgesehen sein, dass beide Gebläse zur Erzeugung des z.B. PEEP beitragen und/oder beide Gebläse in Kombination die hochfrequente Oszillation erzeugen. Beispielsweise kann durch eine gemeinsame Erzeugung der hochfrequenten Oszillation durch beide Gebläse eine höhere Frequenz und/oder Amplitude erreicht werden. Bei zwei Gebläsen, welche eine entgegengesetzte Förderrichtung aufweisen, bremst dabei das eine Gebläse ab während das andere Gebläse beschleunigt, somit wird eine Druckänderung in die gleiche Richtung (höherer oder niedrigerer Druck) erzeugt. Durch die zwei in Reihe geschalteten Gebläse kann somit die Frequenzerzeugung beider Gebläse gebündelt werden. Weist beispielsweise das eine Gebläse eine Beschleunigung von 100mbar/0.1sec auf und das andere Gebläse eine Abbremsung von 100mbar/0.1sec kann eine kombinierte Druckänderung beider Gebläse von 200mbar/0.1sec erreicht werden. Mit dem erfinderischen Beatmungsgerät 1 kann zumindest eine Amplitude der hochfrequenten Oszillation von 50 mbar bei 3 Hz erreicht werden, in manchen Ausführungsformen kann eine Amplitude von 50 mbar bei 5 Hz erreicht werden. In manchen Ausführungsformen kann mindestens eine Amplitude von 100 mbar bei 5 Hz erreicht werden. Bei geringeren Amplituden können in manchen Ausführungsformen auch höhere Frequenzen erreicht werden.

In manchen Ausführungsformen kann ergänzend vorgesehen sein, dass Bypass-Leitungen und Ventile so angeordnet sind, dass beide Gebläse 1010, 1011 in die gleiche Richtung einen Druck aufbauen können. Beispielsweise führt ein Bypass vom Auslass 1014 bis hinter das Gebläse 1010 und ein zweiter Bypass zwischen den Gebläsen 1010, 1011 und hinter das Gebläse 1010. Der zweite Bypass hat dabei endet dabei näher am Anschluss 105 als der erste Bypass. In einer Ventilstellung kann vorgesehen sein, dass sowohl Gebläse 1010 als auch Gebläse 1011 Gas über den Anschluss 105 ansaugen und in Richtung Auslass 1014 fördern. Der Druckaufbau würde dann also in Richtung Auslass 1014 erfolgen. In manchen Ausführungsformen können alternativ oder ergänzend Bypass-Leitungen so angeordnet sein, dass auch ein gemeinsamer Druckaufbau der Gebläse 1010, 1011 in Richtung Anschluss 105 möglich ist.

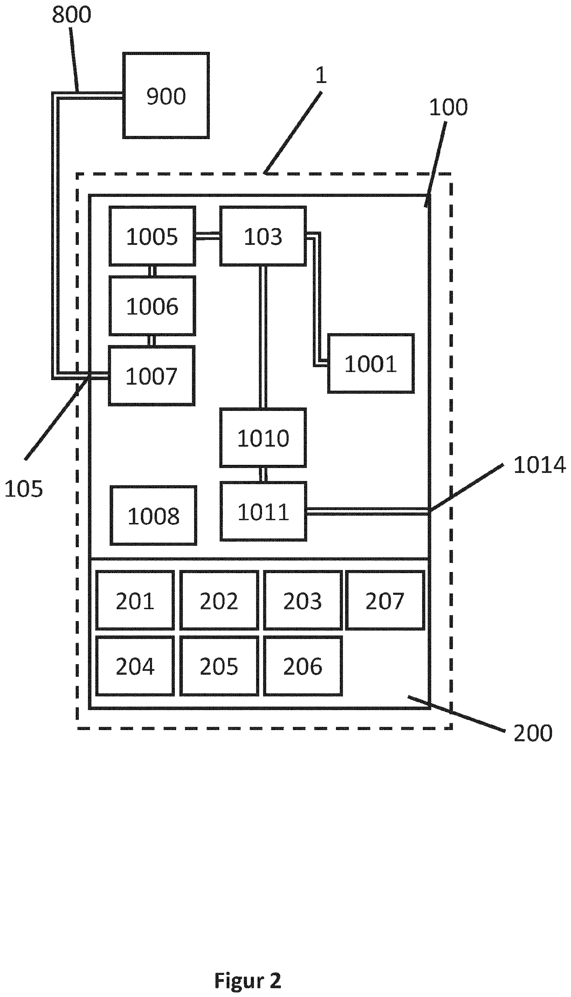

In Figur 2 ist eine weitere beispielhafte Ausführungsform des Beatmungsgerätes 1 schematisch dargestellt. Der Aufbau mit zwei Gebläsen 1010, 1011 wird dabei um zumindest eine Gasquelle 1001 ergänzt. Über die Gasquelle 1001 kann dem geförderten Atemgas zusätzliches Gas zugemischt werden. Beispielsweise kann vorgesehen sein, dass die Gasquelle 1001 als Sauerstoffquelle ausgebildet ist. Über die Sauerstoffquelle kann die Sauerstoffkonzentration des Atemgases eingestellt werden. Das zusätzlich Gas aus der Gasquelle 1001 wird beispielhaft über ein Ventil 103 in die Atemgasleitung des Gasmoduls 100 eingeleitet. Es kann vorgesehen sein, dass im Gasmodul 100 zumindest ein Gassensor angeordnet ist, über welchen die Gaskonzentration des Gases der Gasquelle 1001 im Atemgas bestimmt werden kann.

Zusätzlich kann im Bereich des Ventils 103 auch ein Mischbereich vorgesehen sein, welcher für eine bessere Mischung des Atemgases mit dem aus der Gasquelle 1001 zugesetzten Gas sorgt.

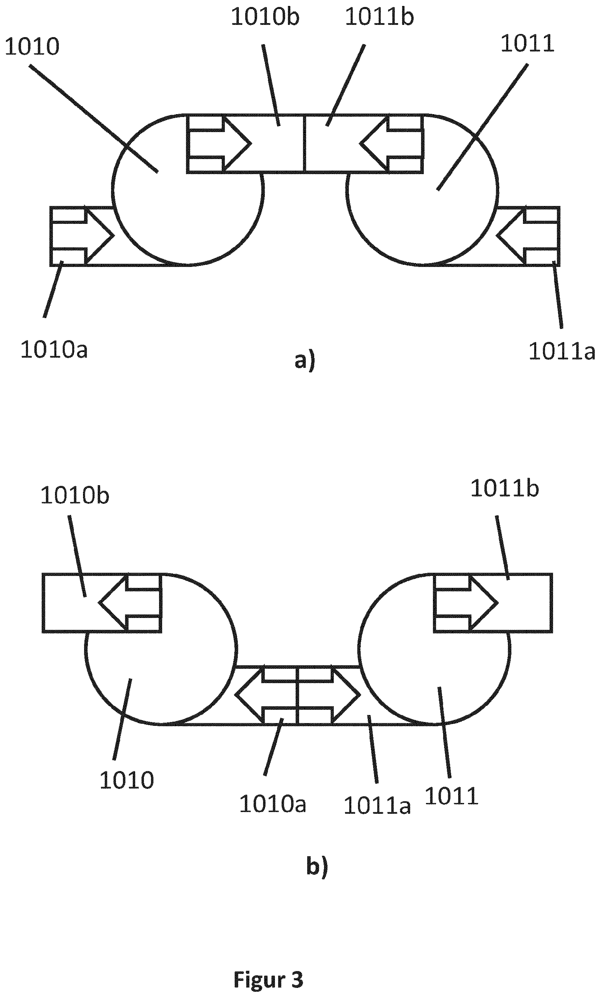

In den Figuren 3 a) und b) sind zwei mögliche Anordnungen der Gebläse 1010, 1011 zueinander schematisch dargestellt. In der Variante aus Figur 3 a) sind die beiden Gebläse 1010, 1011 über die jeweiligen Auslassstutzen 1010b, 1011b miteinander verbunden. Das Gebläse 1010 saugt also über den Ansaugstutzen 1010a Gas an und fördert dies durch den Auslassstutzen 1010b in bzw. durch das Gebläse 1011, soweit der durch das Gebläse 1010 erzeugte Druck den Gegendruck, welcher in manchen Ausführungsformen zumindest teilweise durch das Gebläse 1011 erzeugt wird, überwindet. Soll Gas in die entgegengesetzte Richtung gefördert werden und/oder ein entgegengesetzter Druck erzeugt werden, so ist vorgesehen, dass das Gebläse 1011 einen Druck erzeugt, welcher einen eventuell herrschenden Gegendruck (etwa durch ein langsam laufendes Gebläse 1010) überwindet. Dabei wird durch das Gebläse 1011 Gas durch den Ansaugstutzen 1011a angesaugt und durch den Auslassstutzen 1011b durch das Gebläse 1010 gefördert.

Figur 3 b) zeigt eine umgekehrte Anordnung der Gebläse 1010, 1011. Die Gebläse 1010, 1011 sind dabei über die jeweiligen Ansaugstutzen 1010a, 1011a verbunden. Zur Erzeugung eines Druckes auf der Seite des Auslassstutzen 1010b des Gebläses 1010 wird Gas von dem Gebläse 1010 durch das Gebläse 1011 angesaugt, wobei zumindest ein etwaiger, durch das Gebläse 1011 erzeugter Gegendruck überwunden werden muss. Ebenso verhält es sich, wenn auf Seite des Auslassstutzten 1011b des Gebläses 1011 ein Überdruck erzeugt werden soll, nur das dabei das Gebläse 1011 das Gas durch das Gebläse 1010 ansaugt und einen entsprechenden etwaigen Gegendruck überwinden muss.

### Bezugszeichenliste

1 Beatmungsgerät
100 Gasmodul
103 Ventil
105 Anschluss
200 Steuermodul
201 Steuereinheit
202 Berechnungseinheit
203 Auswerteeinheit
204 Sensoreinheit
205 Eingabeeinheit
206 Speichereinheit
207 Benutzerschnittstelle
800 Schlauchverbindung
900 Patient
1001 Gasquelle
1005 Drucksensor
1006 Flusssensor
1007 Temperatursensor
1008 Drucksensor
1010 Gebläse
1010a Ansaugstutzen
1010b Auslassstutzen
1011 Gebläse
1011a Ansaugstutzen
1011b Auslassstutzen
1014 Auslass

## Patentansprüche

1. Beatmungsgerät (1) zur Beatmung eines Lebewesens umfassend zumindest ein Gasmodul (100) und ein Steuermodul (200), wobei das Gasmodul (100) zumindest zwei Gebläse (1010, 1011) zur Erzeugung eines Überdrucks und/oder eines Unterdrucks und zumindest einen Anschluss (105) zur Verbindung mit einem Schlauch (800) umfasst und das Steuermodul (200) zumindest eine Steuereinheit (201) zur Steuerung des Gasmoduls (100) umfasst, **dadurch gekennzeichnet, dass**
die zumindest zwei Gebläse in Serie geschaltet werden, die Steuereinheit (201) eingerichtet und ausgebildet ist, die Gebläse (1010, 1011) so anzusteuern, dass zumindest ein erstes Gebläse (1010, 1011) eine dem zumindest zweiten Gebläse (1010, 1011) entgegengesetzte Förderrichtung aufweist zumindest ein erstes Gebläse (1010, 1011) so angeordnet ist, dass das Gebläse (1010, 1011) auf der Seite des Anschlusses (105) zumindest zeitweise einen Überdruck erzeugt und
zumindest ein zweites Gebläse (1010, 1011) so angeordnet ist, dass das Gebläse (1010, 1011) auf der Seite des Anschlusses (105) zumindest zeitweise einen Unterdruck erzeugt,
bei der Erzeugung eines Überdrucks auf Seiten des Anschlusses (105) durch zumindest eines der Gebläse (1010, 1011), das zumindest eine andere Gebläse (1010, 1011) mit entgegengesetzter Förderrichtung stillsteht und/oder auf einer Drehzahl läuft, welche geringer ist als die des einen Gebläses (1010, 1011) und/oder einen geringeren Druck erzeugt als das eine Gebläse (1010, 1011),
bei der Erzeugung eines Unterdrucks auf Seiten des Anschlusses (105) durch das andere Gebläse (1010, 1011), das zumindest eine Gebläse (1010, 1011) mit entgegengesetzter Förderrichtung stillsteht und/oder auf einer Drehzahl läuft, welche geringer ist als die des anderen Gebläses (1010, 1011) und/oder einen geringeren Druck erzeugt als das andere Gebläse (1010, 1011).

2. Beatmungsgerät (1) nach Anspruch 1, wobei die zumindest zwei Gebläse (1010, 1011) über ihre Auslassstutzen (1010b, 1011b) miteinander verbunden sind.

3. Beatmungsgerät (1) nach Anspruch 1, wobei die zumindest zwei Gebläse (1010, 1011) über ihre Ansaugstutzen (1010a, 1011a) miteinander verbunden sind.

4. Beatmungsgerät (1) nach zumindest einem der vorhergehenden Ansprüche, wobei das Beatmungsgerät (1) dazu eingerichtet ist, eine Beatmung mit negativem Exspirationsdruck bereitzustellen.

5. Beatmungsgerät (1) nach zumindest einem der vorhergehenden Ansprüche, wobei das Beatmungsgerät (1) dazu eingerichtet ist, einen konstanten Exspirationsfluss zu erzeugen, wobei im Laufe der Exspiration ein über eins der Gebläse (1010, 1011) ein negativer Exspirationsdruck bereitgestellt wird.

6. Beatmungsgerät (1) nach zumindest einem der vorhergehenden Ansprüche, wobei das Beatmungsgerät (1) dazu eingerichtet ist, eine Hustentherapie durchzuführen, wobei einem Patienten (900) während der Inspiration durch das eine Gebläse (1010) ein Atemgas mit Überdruck bereitgestellt wird und nach dem Ende der Inspiration ein schnelles Umschalten zu einem, durch das andere Gebläse (1011) zur Verfügung gestellte, Atemgas mit Unterdruck gewechselt wird.

7. Beatmungsgerät (1) nach zumindest einem der vorhergehenden Ansprüche, wobei das Beatmungsgerät (1) dazu eingerichtet ist, eine überlagerte und/oder oszillierende Beatmung bereitzustellen.

8. Beatmungsgerät (1) nach zumindest einem der vorhergehenden Ansprüche, wobei das eine Gebläse (1010) die Beatmungsfrequenz bestimmt und das andere Gebläse (1011) die überlagerte Frequenz erzeugt.

9. Beatmungsgerät (1) nach zumindest einem der vorhergehenden Ansprüche, wobei das Beatmungsgerät (1) dazu eingerichtet ist, gleichzeitig eine überlagerte Beatmung und eine Beatmung mit negativem Exspirationsdruck bereitzustellen.

10. Beatmungsgerät (1) nach zumindest einem der vorhergehenden Ansprüche, wobei zwischen dem Anschluss (105) und den Gebläsen (1010, 1011) zumindest eine Gasquelle (1001) angeordnet ist, wobei zumindest ein Gas aus der Gasquelle (1001) über zumindest ein Ventil (103) in das durch eins der Gebläse (1010, 1011) geförderte Atemgas eingeleitet wird.

11. Beatmungsgerät (1) nach zumindest einem der vorhergehenden Ansprüche, wobei im Bereich des Ventils (103) ein Mischbereich zur besseren Durchmischung von gefördertem Atemgas und durch das Ventil (103) eingeleitete Gas angeordnet ist.

## Claims

1. A ventilator (1) for ventilating a living being, comprising at least one gas module (100) and one control module (200), wherein the gas module (100) comprises at least two blowers (1010, 1011) for generating a positive pressure and/or a negative pressure and at least one connection (105) for connecting to a hose (800), and the control module (200) comprises at least one control unit (201) for controlling the gas module (100), **characterized in that** the at least two blowers are connected in series, the control unit (201) is configured and designed to actuate the blowers (1010, 1011) such that at least one first blower (1010, 1011) has a conveying direction opposite to the at least second blower (1010, 1011),
at least one first blower (1010, 1011) is arranged such that the blower (1010, 1011) at least temporarily generates a positive pressure on the side of the connection (105) and
at least one second blower (1010, 1011) is arranged such that the blower (1010, 1011) at least temporarily generates a negative pressure on the side of the connection (105),
when a positive pressure is generated on the side of the connection (105) by at least one of the blowers (1010, 1011), the at least one other blower (1010, 1011) with an opposite conveying direction is idle and/or runs at a rotational speed that is lower than that of the one blower (1010, 1011) and/or generates a lower pressure than the one blower (1010, 1011),
when a negative pressure is generated on the side of the connection (105) by the other blower (1010, 1011), the at least one other blower (1010, 1011) with an opposite conveying direction is idle and/or runs at a rotational speed that is lower than that of the other blower (1010, 1011) and/or generates a lower pressure than the other blower (1010, 1011).

2. The ventilator (1) according to claim 1, wherein the at least two blowers (1010, 1011) are connected to each other by their outlet fittings (1010b, 1011b).

3. The ventilator (1) according to claim 1, wherein the at least two blowers (1010, 1011) are connected to each other by their intake fittings (1010a, 1011a).

4. The ventilator (1) according to at least one of the preceding claims, wherein the ventilator (1) is configured to provide ventilation with negative expiratory pressure.

5. The ventilator (1) according to at least one of the preceding claims, wherein the ventilator (1) is configured to generate a constant expiratory flow, wherein, over the course of expiration, a negative expiratory pressure is provided by one of the blowers (1010, 1011).

6. The ventilator (1) according to at least one of the preceding claims, wherein the ventilator (1) is configured to perform cough therapy, wherein a patient (900) is provided with respiratory gas with positive pressure by one blower (1010) during inspiration and, after the end of inspiration, a fast switch to a respiratory gas with negative pressure provided by the other blower (1011) is carried out.

7. The ventilator (1) according to at least one of the preceding claims, wherein the ventilator (1) is configured to provide superimposed and/or oscillatory ventilation.

8. The ventilator (1) according to at least one of the preceding claims, wherein one blower (1010) determines the ventilation frequency and the other blower (1011) generates the superimposed frequency.

9. The ventilator (1) according to at least one of the preceding claims, wherein the ventilator (1) is configured to simultaneously provide superimposed ventilation and ventilation with negative expiratory pressure.

10. The ventilator (1) according to at least one of the preceding claims, wherein at least one gas source (1001) is arranged between the connection (105) and the blowers (1010, 1011), wherein at least one gas from the gas source (1001) is introduced into the respiratory gas conveyed by one of the fans (1010, 1011) via at least one valve (103).

11. The ventilator (1) according to at least one of the preceding claims, wherein a mixing region for better mixing of conveyed respiratory gas and gas introduced through the valve (103) is arranged in the region of the valve (103).

## Revendications

1. Appareil respiratoire (1) pour l'assistance respiratoire d'un être vivant, comportant au moins un module de gaz (100) et un module de commande (200), dans lequel le module de gaz (100) comporte au moins deux ventilateurs (1010, 1011) destinés à produire une pression positive et/ou une pression négative et au moins un raccord (105) destiné au raccordement avec un tube (800) et le module de commande (200) comporte au moins une unité de commande (201) destinée à commander le module de gaz (100), **caractérisé en ce que** les au moins deux ventilateurs sont montés en série, l'unité de commande (201) est configurée et réalisée pour actionner les ventilateurs (1010, 1011) de telle façon qu'au moins un premier ventilateur (1010, 1011) présente une direction de transport opposée à l'au moins deuxième ventilateur (1010, 1011),
au moins un premier ventilateur (1010, 1011) est disposé de telle façon que le ventilateur (1010, 1011) produit au moins temporairement une pression positive sur le côté du raccord (105) et
au moins un deuxième ventilateur (1010, 1011) est disposé de telle façon que le ventilateur (1010, 1011) produit au moins temporairement une pression négative sur le côté du raccord (105),
lors de la production d'une pression positive du côté du raccord (105) par l'un au moins des ventilateurs (1010, 1011), l'au moins un autre ventilateur (1010, 1011) présentant la direction de transport opposée est à l'arrêt et/ou fonctionne à une vitesse inférieure à celle dudit ventilateur (1010, 1011) et/ou produit une pression inférieure par rapport audit ventilateur (1010, 1011),
lors de la production d'une pression négative du côté du raccord (105) par l'autre ventilateur (1010, 1011), l'au moins un autre ventilateur (1010, 1011) présentant la direction de transport opposée est à l'arrêt et/ou fonctionne à une vitesse inférieure à celle de l'autre ventilateur (1010, 1011) et/ou produit une pression inférieure par rapport à l'autre ventilateur (1010, 1011).

2. Appareil respiratoire (1) selon la revendication 1, dans lequel les au moins deux ventilateurs (1010, 1011) sont reliés entre eux par le biais de leurs tubulures de sortie (1010b, 1011b).

3. Appareil respiratoire (1) selon la revendication 1, dans lequel les au moins deux ventilateurs (1010, 1011) sont reliés entre eux par le biais de leurs tubulures d'aspiration (1010a, 1011a).

4. Appareil respiratoire (1) selon l'une au moins des revendications précédentes, dans lequel l'appareil respiratoire (1) est configuré pour fournir une assistance respiratoire avec une pression d'expiration négative.

5. Appareil respiratoire (1) selon l'une au moins des revendications précédentes, dans lequel l'appareil respiratoire (1) est configuré pour produire un flux d'expiration constant, dans lequel une pression d'expiration négative est fournie par l'un des ventilateurs (1010, 1011) au cours de l'expiration.

6. Appareil respiratoire (1) selon l'une au moins des revendications précédentes, dans lequel l'appareil respiratoire (1) est configuré pour effectuer un traitement de la toux, dans lequel un gaz respiratoire présentant une pression positive est fourni à un patient (900) par un des ventilateur (1010) pendant l'inspiration et une commutation rapide sur un gaz respiratoire présentant une pression négative, fourni par l'autre ventilateur (1011) est effectuée à la fin de l'inspiration.

7. Appareil respiratoire (1) selon l'une au moins des revendications précédentes, dans lequel l'appareil respiratoire (1) est configuré pour fournir une assistance respiratoire superposée et/ou oscillante.

8. Appareil respiratoire (1) selon l'une au moins des revendications précédentes, dans lequel un ventilateur (1010) détermine la fréquence respiratoire et l'autre ventilateur (1011) produit la fréquence superposée.

9. Appareil respiratoire (1) selon l'une au moins des revendications précédentes, dans lequel l'appareil respiratoire (1) est configuré pour fournir simultanément une assistance respiratoire superposée et une assistance respiratoire présentant une pression d'expiration négative.

10. Appareil respiratoire (1) selon l'une au moins des revendications précédentes, dans lequel au moins une source de gaz (1001) est disposée entre le raccord (105) et les ventilateurs (1010, 1011), dans lequel au moins un gaz est introduit à partir de la source de gaz (1001) par le biais d'au moins une soupape (103) dans le gaz respiratoire transporté par l'un des ventilateurs (1010, 1011).

11. Appareil respiratoire (1) selon l'une au moins des revendications précédentes, dans lequel un zone de mélange est disposée dans la zone de la soupape (103) pour améliorer le mélange de gaz respiratoire transporté et de gaz introduit par la soupape (103).
